(19) **Europäisches Patentamt / European Patent Office / Office européen des brevets**

(11) **EP 4 585 182 A1**

(12) # EUROPEAN PATENT APPLICATION

(43) Date of publication:
**16.07.2025 Bulletin 2025/29**

(21) Application number: **24150836.5**

(22) Date of filing: **09.01.2024**

(51) International Patent Classification (IPC):
**A61C 1/00** *(2006.01)*      **A61B 18/20** *(2006.01)*
**A61C 19/06** *(2006.01)*      **A61N 5/06** *(2006.01)*

(52) Cooperative Patent Classification (CPC):
**A61C 19/06; A61B 5/0088; A61N 5/0603;**
A61C 1/052; A61C 17/22; A61N 2005/007;
A61N 2005/0606

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **FRACKOWIAK, Bruno Jean François**
**Eindhoven (NL)**

• **HOETZL, Sandra**
**Eindhoven (NL)**
• **VAN DEN DUNGEN, Wilhelmus Andreas Marinus Arnoldus Maria**
**Eindhoven (NL)**
• **PAULUSSEN, Elvira Johanna Maria**
**Eindhoven (NL)**
• **GOTTENBOS, Bart**
**Eindhoven (NL)**

(74) Representative: **Philips Intellectual Property & Standards**
**High Tech Campus 52**
**5656 AG Eindhoven (NL)**

(54) **HEAD COMPONENT FOR AN ORAL CARE PRODUCT**

(57) Proposed is a head component for an oral care product. In particular, it is proposed to adapt a head component to include both a light emitting structure, to target a particular area of a subject's oral surfaces with a light treatment, and a fluid-based cooling system to extract heat from the targeted area.

**FIG. 1A**

**(Cont. next page)**

**EP 4 585 182 A1**

**FIG. 1B**

**Description**

FIELD OF THE INVENTION

The present invention relates to the field of oral care products, and more particularly to a head component for an oral care product, such as an oral irrigator or mouthpiece.

BACKGROUND OF THE INVENTION

Oral care products with light treatment capabilities have been shown to have many oral health benefits. For example, light treatments may provide antimicrobial effects, anti-inflammatory effects, stain removal capabilities, and an extension of whitening benefits, all of which may be useful tools in an oral health care routine. Light treatments have the additional advantage that they may be able to target areas which are difficult to reach with mechanical brushing e.g., areas around the gumline or interdental cavities.

To achieve these benefits, the light treatment time is required to be at least tens of seconds to even minutes. Illuminating the treatment area with a higher intensity may help to achieve shorter treatment times. A proportion of the light incident on the target area will be absorbed and converted to heat, the higher the intensity of the incident light the greater the level of heating. Additionally, light sources used for such purposes have a wall plug efficiency less than 100% meaning that higher illumination levels will result in larger amounts of energy dissipated as heat. If the light source is too close to the target area of the user's teeth and gums, this dissipated heat will add to the heat induced by absorption of light at the teeth or gum tissue. This may result in the temperature of biological tissues of the user increasing above a safety limit without achieving a sufficient irradiance level for efficient light treatment of the target area.

SUMMARY OF THE INVENTION

The invention is defined by the claims.

According to examples in accordance with an aspect of the invention, there is provided a head component for an oral care product for insertion into a subject's mouth during use, wherein the head component comprises:

   a light emitting structure configured to emit light to a target area of an oral surface of the subject when in use; and
   a fluid-based cooling system configured to establish a fluid flow that extracts heat from the target area.

Proposed are schemes, solutions, concepts, designs, methods and systems pertaining to aiding and/or improving a head component for an oral care product.

In particular, it is proposed to adapt a head component to include both a light emitting structure, to target a particular area of the subject's teeth and gums with a light treatment, and a fluid-based cooling system to extract heat from the targeted area. To achieve oral health care benefits from light treatment procedures, the intensity of light incident on a targeted area is required to be above a minimum level, especially if the treatment time is to be kept relatively short. At these intensities, the absorption of light and the dissipation of this energy as heat at the target area may lead to significant local heating. The light emitting structure itself will also dissipate heat due to its less than 100% efficiency. In a direct illumination design wherein the light emitting structure is positioned in the close vicinity of the target area to achieve the most efficient transmission of light, this heat may be absorbed by the tissues of the target area leading to further temperature increases. Heating of the gums and other oral surfaces of a subject above a recommended level may result in the subject experiencing pain and discomfort and may even lead to damage. The presence of a fluid-based cooling system may therefore be advantageous in extracting heat from the target area such that high light intensities can be used for effective treatment, without dangerous heating of the subject's critical tissues.

For example, a fluid-based cooling system implemented in the proposed invention may comprise a nozzle configured to emit fluid to the target area and thereby provide means for extracting heat from the target area. The emitted fluid may also act in a cleaning capacity providing further oral health benefits. Another possible solution is the provision of fluid channel to direct a fluid flow along a path adjacent to the target area to provide continuous cooling.

A further advantage of a fluid-based cooling system is that the fluid flow established may act as a light guide. The refractive index of the fluid flow and the medium surrounding the fluid flow may be configured such that light incident on the fluid flow at an angle to the direction of flow is internally reflected at the fluid-medium interface. In this manner the light may be channelled within the fluid flow and focused on the target area. This may result in more efficient transmission of light from the light emitting structure to the target area and therefore a lower emitted intensity is required to achieve the same incident intensity on the target area, reducing the heat dissipated by the light emitting structure.

It was therefore realised that by combining light treatment capabilities with a fluid-based cooling system the temperature increase of critical tissues may be reduced and the optical coupling between the light source and the targeted area may be improved to ensure sufficient illumination for treatment purposes.

An improved head component for an oral care product for insertion into a subject's mouth during use may thus be supported by the proposed concept(s).

In some embodiments, the fluid-based cooling system may comprise a nozzle configured, in use, to emit a jet of fluid to the target area, and wherein the fluid flow may comprise the jet of cleaning fluid. Fluid jets have been proven to be effective at mechanically removing biofilms from oral surfaces and may in the proposed invention provide the additional benefit of inducing cooling of the target area. Fluid jets may also be able to act as light guides to the light emitted by the light emitting structure and thereby enhance the illumination of the target area.

In some embodiments, the light emitting structure may be embedded within the nozzle. This is a useful configuration to ensure that the fluid emitted by the nozzle targets the same area as the light emitting structure and provides efficient light guidance capabilities.

In some embodiments, the light emitting structure may be located behind the nozzle and the nozzle may be transparent. This configuration allows for the light emitting structure and the nozzle to be separate components whilst ensuring emission towards the same target area.

In some embodiments, the fluid-based cooling system may comprise a fluid channel configured to direct the fluid flow. A closed or open channel arrangement may be used to direct a fluid flow close to the target area such that the fluid flow extracts heat from the target area.

In some embodiments, the fluid channel may be configured to be adjacent to the teeth and gums of the user when in use. Heat may then be transferred from the teeth and gums of the user to the fluid flow in the fluid channel via the walls of the fluid channel. The fluid flow may then extract the heat to an area sufficiently far away from the target area.

In some embodiments, the fluid channel may be configured to be adjacent to the light emitting structure so that the fluid-based cooling system is further adapted to extract heat from the light-emitting structure. This may aid in removing the heat dissipated by the light emitting structure away from the target area, further reducing the temperature increase of the target area during the light treatment.

In some embodiments, the fluid channel may be configured to transport the fluid flow to a nozzle for emission to the oral surface of the subject. In this way, the target area may be provided with both indirect cooling via the fluid channel, and direct cooling via the emission of fluid to the oral surface.

In some embodiments, the fluid channel may comprise a close re-circulation loop within the head component. This allows for cooling to still be active even if there are no jets exiting from the head component. The closed loop arrangement further limits the amount of wasted fluid and allows for the possible usage of a more suitable cooling fluid.

In some embodiments, the light emitting structure may emit at least one of: blue light; violet light; infrared light; and ultraviolent light. Such forms of light have all been proven to have oral hygiene benefits,

In some embodiments, the head component may further comprise a light sensor configured to detect light reflected off the target area of the subject when the device is in use. The reflected light may be indicative of which area of the user's teeth and gums that is being targeted by the light emitting device and may further include oral health and hygiene information.

According to another aspect of the invention there is provided an oral care product comprising a head component according to any proposed embodiment. For example, the oral care product may comprise an electric toothbrush, an oral irrigator, or a mouthpiece device.

In some embodiments, the oral care product may further comprise: a fluid reservoir; and a pump configured to pump fluid from the fluid reservoir to the fluid-based cooling system. In this way, the system may be provided with means of providing fluid to the fluid reservoir in a handheld device.

In some embodiments, the oral care product may further comprise: a handle configured to attach to the head component; a light emitting device situated in the handle; and a light guide tube configured to transport light from the light emitting device to the light emitting structure of the head component. This may allow for illumination of the target area without the need for the light emitting device to be adjacent to the user's teeth and gums. This may further provide cooling of the target area. Optionally the light guide tube may comprise the fluid-based cooling system of the head component.

In some embodiments, the oral care product may further comprise a light sensor of the head component configured to generate a light detection signal describing the light reflected off the target area of the subject when the system is in use; and a processing arrangement configured to: receive the light detection signal from the light sensor; and based on the light detection signal, control the light emitted by the light emitting structure of the head component. The presence of a light sensor and the analysis of a light detecting signal from it may allow for determination of the form of the oral surface that is being targeted by the light emitting device. This information may be used to control the product to operate in different treatment modes that may be suited to different oral surfaces.

Further, controlling the light emitted by the light emitting structure may comprise at least one of: controlling the intensity of the light emitted; controlling the power of the light emitted; and controlling the wavelength of the light emitted. In this way the irradiance and dosage received by the target area may be controlled to suit the specific needs of the subject.

These and other aspects of the invention will be apparent form and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1A is a simplified block diagram of a portion of a head component for an oral care product for insertion into a subject's mouth during use according to a proposed embodiment;
Fig. 1B is a simplified block diagram of a portion of a head component for an oral care product for insertion into a subject's mouth during use according to another proposed embodiment;
Fig. 2 depicts a fluid flow acting as a light guide;
Fig. 3 is a simplified block diagram of a mouthpiece device for insertion into a subject's mouth during use according to a proposed embodiment;
Fig. 4 is a simplified block diagram of oral care product according to a proposed embodiment;
Fig. 5 is a simplified block diagram of a portion of a head component for an oral care product according to an alternative embodiment; and
Fig. 6 is a simplified block diagram of an oral care product according to another proposed embodiment.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various arrangements, adaptions, and/or configurations of a head for an oral hygiene product pertaining to improving cleaning performance. According to proposed concepts, a number of possible solutions may be implemented separately or jointly. That is, although these possible solutions may be described below separately, two or more of these possible solutions may be implemented in one combination or another.

The invention proposes concepts for aiding and/or improving cleaning of an oral care product for insertion into a subject's mouth during use. In particular, embodiments may provide a system, device and/or method which provides both a light emitting structure, to emit light to a target area of an oral surface of the subject, and a fluid-based cooling system, configured to extract heat from the target area. This configuration permits a high intensity of light to be incident on the target area, which may be beneficial to improving the effectiveness of the light treatment, whilst preventing dangerous overheating. Put another way, the fluid-based cooling system may help to extract heat absorbed by the target area so as to prevent overheating.

The provision of a fluid-based cooling system, alongside light treatment capabilities, may permit safe application of light to a user's oral surfaces. A proportion of the light incident on the oral surfaces of the user during a light treatment will be absorbed and dissipated as heat. This may result in a localised temperature increase at the targeted area. Further, light emitting structures have a certain wall plug efficiency (e.g., 45%) which results in a large fraction of their total power being dissipated as heat. If the light emitting structures are positioned proximate to the area being targeted by the treatment (as is optimal for the most efficient transmission of light to the area), this may result in further heating of the user's oral surfaces. The effect of this combined heating may result in a temperature increase in the biological tissues that is above a safety limit (for example, for pain receptors located 0.2 mm below the gum surface it is deemed unsafe if the temperature rises above 43 °C). This may lead to user discomfort or even damage to the oral surface. The presence of a fluid-based cooling system to extract heat from the target area may enable high light intensities to be used without risk to the user.

A fluid-based cooling system may provide the additional advantage of light guiding. If the optical properties of the fluid used within the fluid-based cooling system are similar to those of the target tissue, the fluid flow may improve the coupling between the light emitting structure and the target area. Light emitted by the light emitting structure may be focussed and directed by the fluid flow to result in more efficient light transport and less reflection at the target surface. This may enable higher intensity illumination to be achieved for the same light source power. Since the effectiveness of the light treatment is directly related to the intensity of the incident light, this property may result in more effective treatment of the target area.

The proposed concepts may, for example, be applied to oral irrigators, electric toothbrushes, and mouthpiece-style devices. In other words, proposed concepts may be incorporated into a wide range of oral care products to enhance light treatment in a safe and sustainable manner.

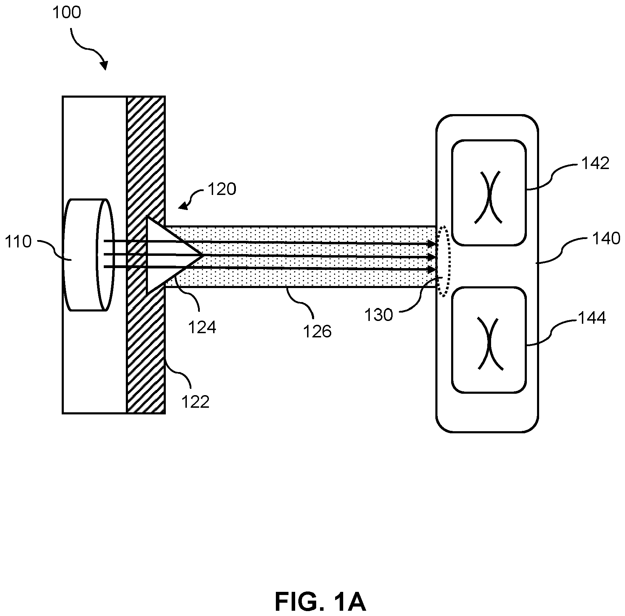

Referring to Fig. 1A, there is shown a simplified block diagram of a portion of a head component 100 for an oral care device for insertion into a subject's mouth during use according to a proposed embodiment.

The head component 100 comprises a light emitting structure 110 configured to emit light (indicated by the straight arrows in Fig. 1A) to a target area 130 of an oral surface of the user. In this exemplary embodiment, the target area is a portion of the gums 140 neighbouring the two teeth 142 and 144. The light emitting structure 110 in this exemplary embodiment is an LED configured to emit blue light (blue light has been proven to have antibacterial and anti-inflammatory benefits whilst having low absorbance and therefore results in less heating).

The head component 100 further comprises a fluid-based cooling system 120 configured to establish a fluid flow to extract heat from the target area 130. In this exemplary embodiment, the fluid-based cooling system comprises a fluid channel 122 configured to direct the fluid flow and transport it to a nozzle 124. The nozzle 124 is further configured to emit a jet of fluid 126 to the target area 130. In this example the fluid flow comprises both the fluid contained within the fluid channel 122 and the fluid within the jet of fluid 126. In this embodiment the fluid of both the jet of fluid 126 and within the fluid channel 122 is water, however any suitable cooling fluid may be used. Jets of water and other suitable cleaning fluids (e.g., mouthwashes) have been shown to be beneficial in oral care products by removing biofilms in areas which are difficult to reach with a regular toothbrush. Therefore, the jet of fluid 126 may aid in both cleaning and cooling the target area.

Fluid incident on the target area from the jet of fluid, may splatter off the oral surface of the user in different directions. In this way the splattered jets may carry away residual heat form the target area to less critical areas and/or spread the dissipated heat energy over a larger volume. In this way, the fluid jet may induce cooling of the target area. The jet of fluid must be sufficiently powerful to generate the mechanical action on the target area required for biofilm removal and cleaning, whilst providing a sufficiently high fluid flow rate to efficiently carry heat away from the target area.

The fluid flow contained within the fluid channel 122 may further aid cooling of the target region. Since the fluid channel 122 is configured to be adjacent to the light-emitting structure 110, the fluid channel 122 may extract dissipated heat from the light emitting device which occurs due to the less than 100% efficiency of the light emitting structure.

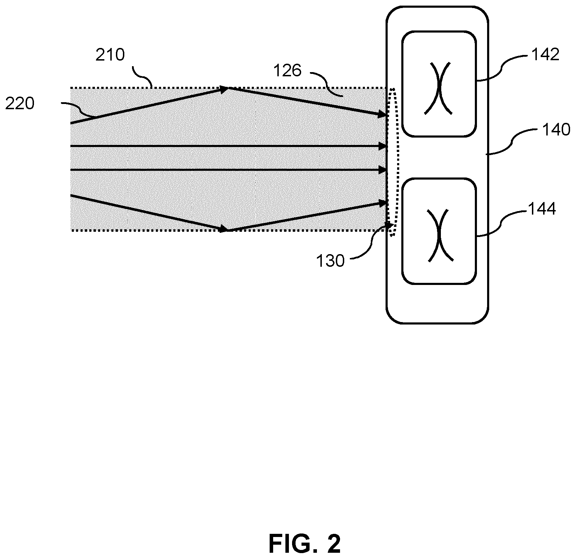

In this exemplary embodiment the light emitting structure 110 is placed behind the nozzle 124 and the nozzle 124 is transparent. This enables the jet of fluid to be directed along substantially the same axis as the light emitted by the light emitting structure, ensuring that both target the same area of the user's teeth and gums, thereby permitting a maximum cooling effect. A further advantage of this configuration is that the jet of fluid may act as a light guide, guiding the light emitted by the light emitting structure towards the target area. This may enhance the illumination in the target area while limiting light losses. Further details of this process are given in the discussion of Fig. 2 below.

Although in the exemplary head component 100, the light-emitting structure is a blue light LED, in alternative embodiments the light-emitting structure may be any suitable light emitting device, preferably a device with a high efficiency. The light-emitting structure may be configured to emit at least one of: blue light; violet light; infrared light; and ultraviolet light, which have all been proven to provide oral health care benefits. The wavelength of light used may depend on the oral care benefit required.

Similarly, other aspects of head component 100 may be implemented differently in alternative embodiments. For example, the light emitting structure 110 and the nozzle 124 may be integrated into the same structure such that the light emitting structure is embedded within the nozzle this may save space within the head component. Alternatively, the fluid-based cooling system may not comprise the nozzle 124 at all. The fluid channel in this instance be a closed re-circulation loop in which fluid is passed repeatedly past the target area. In Fig. 1A the light emitting structure is depicted as being located behind the fluid-based cooling system, in other embodiments the light emitting structure may be in front of the fluid-based cooling system. This configuration has the advantage that when the head component is in use, the light emitting structure is located closer to the target area and therefore a higher treatment intensity may be able to be achieved. The target area may be a portion of any oral surface, for example the teeth, gums, tongue, cheeks, or interdental space.

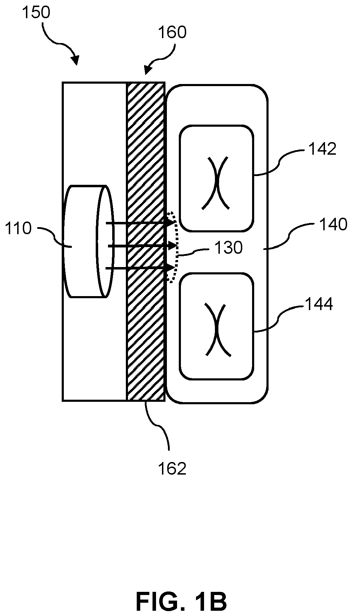

Referring now to Fig. 1B, there is depicted a simplified block diagram of a portion of a head component 150 for an oral care product for insertion into a subject's mouth during use according to another proposed embodiment. The head component 150 comprises the light emitting structure 110, which may be identical to the light emitting structure 110 of the head component 100, configured to emit light to the target area 130 of an oral surface of the subject when in use. The head component 150 further comprises a fluid-based cooling system 160 configured to establish a fluid flow that extracts heat from the target area. The fluid-based cooling system 160 comprises a fluid channel 162. A fluid flow is established within the fluid channel 162 that extracts heat from critical areas of the user's teeth and/or gums. The fluid used within the fluid-based cooling system 160 may comprise water, oil, or any other suitable cooling fluid.

The fluid channel 162 is configured to be adjacent to the teeth and gums of the user and may be further configured to contact at least a portion of the user's oral surfaces as shown in Fig. 1B. Configuration of the fluid channel in this way enables heat to be extracted directly from the oral surface of the user through the walls of the fluid channel 162.

In this exemplary embodiment, the fluid channel 162 comprises a flexible transparent rubber tube that may deform to the shape of the user's teeth and gums, although the present invention is not limited to such. The flexibility of the fluid channel 162 enables the contact area between the fluid channel 162 and the oral surfaces of the user to be maximised and therefore improves the efficiency of heat transfer. The transparency of the fluid channel 162 may be beneficial for providing light guidance capabilities as discussed below in reference to Fig. 2.

Since the fluid channel 162 is configured to be adjacent to both the target area 130 and the light emitting structure 110, the fluid flow within the fluid channel 162 is adapted to extract heat both from the target area and from the light emitting structure. This is advantageous as cooling of the target area is achieved by both direct heat extraction and by reducing the amount of heat incident on the target area from the light emitting structure.

Referring now to Fig. 2, there is depicted a fluid flow acting as a light guide. This figure shows in more detail the interaction between the light emitted by the light emitting structure and the jet of fluid 126. An additional benefit of the use of a fluid-based cooling system in conjunction with light treatment, is that the fluid-based cooling system may act as a light guide to target the emitted light towards the desired target area. Fig. 2 shoes how light 220 emitted from a light emitting structure may be internally reflected at a fluid interface 210 such that the light remains within the boundaries of the fluid flow. In this way, the fluid flow may be used to direct the light emitted by the light emitting structure. This may lead to a higher proportion of the light that is emitted reaching the intended target area 130.

In this example, we consider the case when the fluid-based cooling system comprises the water jet 126 directed towards a target area 130. Water exhibits optical properties (a refractive index $n \approx 1.33$) which are very close to those of biological tissues (for example, the refractive index of gum tissue is $n \approx 1.43$), and different from those of air ($n = 1$). Therefore, when water is used in a jet, at the interface between the biological tissue of the target area and the water jet, light transmission is maximised. On the other hand, at the water/air interface at the boundary of the jet, light reflection is maximised and therefore most of the incident water is reflected back inside the jet. This enhances the intensity of light absorbed by the target area compared to the case in which no jet is used and there is a volume of air between the light emitting structure and the target area.

In alternative embodiments, a fluid channel may also be configured to have light guiding capabilities. A fluid channel may comprise a soft transparent rubber tube that is permitted to deform around the gum and teeth anatomy of the user (as shown in Fig. 1B), to ensure maximal coupling between the fluid channel and the user's oral surfaces. By using a material for the fluid channel that is transparent and has a refractive index close to that of the biological tissues of the user, the light transmission between the tube and the oral surfaces of the user may be maximised if the tube is in contact with the teeth and/or gums of the user, whereas the light will be internally reflected in areas where the tube is surrounded by air. In this way, the rubber fluid channel will transmit light to the user on its contacting part and act as a light guide on the remaining part surrounded by air.

As an example, for a direct illumination design in which the light emitting structure is placed proximate to the target area and is not provided with a cooling system, typically an irradiance of 300 mW/cm$^2$ over a 15 s exposure time may be used. With these settings, the dose received by the user is sufficiently high to achieve a reduction in plaque regrowth and other such oral health benefits, without overheating the tissue. When using a fluid-based cooling system with light guiding capabilities, the same light source may be focused on a 15 times smaller surface area, creating an irradiance of 4.5 W/cm$^2$. At this exposure level, the same dose can be achieved in a 1 s exposure time. However, this level of irradiance may ordinarily lead to overheating of critical tissues.

Considering a typical water jet present in an oral irrigator product (with ~ 1 mm diameter and an ~1 m/s velocity), the cooling effect of the water jet may be quantified. The maximal cooling heat flux that can be extracted by the jet is given by

$$Q_{max} = h(T_S - T_{amb}) \approx 17 \, \mathrm{Wcm^{-2}},$$

where $h$ is the heat transfer coefficient ($h = 2.82$ W/K/cm$^2$), $T_S$ is the temperature of the tissue surface (which we take to be equal to the thermal safety limit 43 °C), and $T_{amb}$ is the ambient temperature (taken to be 37 °C). Therefore, since the maximal cooling heat flux of the jet is above the focused light irradiance of 4.5 W/cm$^2$, it is possible with this set up to cool the tissue to temperatures below the safety limit. This shows the feasibility of the use of a water jet as part of a fluid-based cooling system.

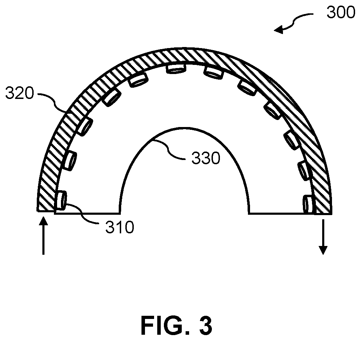

Referring now to Fig. 3, there is depicted a simplified block diagram of a mouthpiece device for insertion into a subject's mouth during use according to a proposed embodiment.

The head component of the present invention may be adapted for a variety of different oral care products. Implementing the head component as a mouthpiece may have the additional advantage of reducing the total treatment time required as multiple oral surfaces of the user may be treated at once. The mouthpiece 300 comprises a dental tray 330 configured to mimic the dental arch of a subject and receive of a portion of the subject's teeth and/or gums.

The mouthpiece further comprises light emitting devices 310 which are configured, once the device has been inserted into the subject's mouth, to emit light to target areas of at least one oral surface of the subject's mouth. For example, the light emitting structures may be positioned within the mouthpiece device so that they target the interdental spaces of the subject. Other positions and configurations of the light emitting devices may be advantageous depending on the type of treatment required.

The mouthpiece device 300 further comprises a fluid channel 320 configured to be adjacent to the light emitting structure 310 and the teeth and gums of the subject when the device is in use. The fluid channel 320 contains a fluid flow that is directed around the contour of the mouthpiece (as indicated by the arrows in Fig. 3) and thereby absorbs and extracts heat from the target areas of the teeth and gums of the subject and from the light emitting devices 320.

In this exemplary embodiment the fluid channel comprises a closed recirculation loop. By this it is meant that the fluid that forms the fluid flow contained within the fluid channel is not emitted to the teeth and gums of the user but remains within the head component or the system that contains the head component. The fluid channel 320 may be connected to a fluid reservoir (not shown) that is within or external to the mouthpiece device 300. Fluid may be configured to flow from the fluid reservoir to the fluid channel of the mouthpiece, around the arc of the mouthpiece and then back to the reservoir. Heat is carried away from the from the target areas of the mouth to the fluid reservoir by this flow. Effective use of this configuration requires the quantity of water in fluid reservoir to be sufficient such that the temperature increase that occurs as the water passes the target areas is negligible.

An alternative solution is a fluid flow that circulates within the fluid channel of the mouthpiece only. In this instance, fluid that has been heated by the light emitting structure and the target area will cool off in areas sufficiently far away from critical oral surfaces such as the gums. This may be less efficient than a configuration in which the fluid is directed to a fluid reservoir but may be sufficient to keep the temperature of the critical tissue within a safe limit if the treatment time is not too long. Closed recirculation loop configurations limit the amount of wasted fluid used for thermal regulation and allow for a wider range of fluids to be used in the cooling system. In open configurations in which the fluid is emitted to the teeth and/or gums of the subject, the fluid must be safe and suitable for cleaning purposes. In a closed loop design, other liquids such as oil may be used which may provide enhanced cooling capabilities.

In alternative embodiments, a mouthpiece of the proposed invention may further comprise brushing elements for manual or automatic cleaning of the teeth. This may require there to be a gap between the light emitting structures embedded in the mouthpiece and the teeth and/or gums of the user to ensure sufficient room for the brushing elements. In this case, the presence of jets may be beneficial to ensure efficient coupling between the light emission and the targeted area, as the air layer that is otherwise present results in large light losses as light is reflected at the air/tissue boundary. The jet may additionally improve the light coupling to the target area by removing toothpaste or other obstructions from the light path.

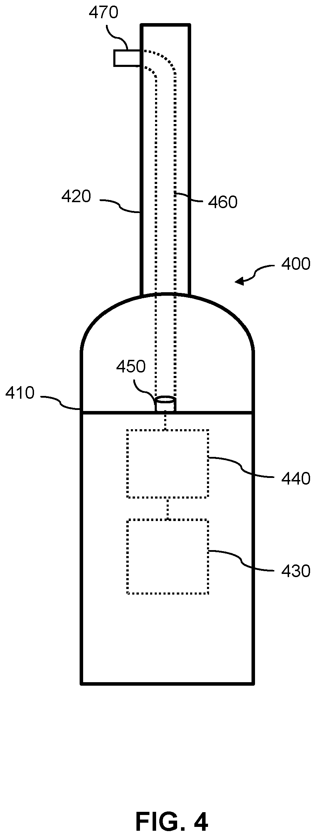

Referring now to Fig. 4, there is depicted a simplified block diagram of an oral care product 400.

In this example, the oral care product 400 is an oral irrigator device. The oral irrigator 400 comprises a handle 410 configured to attach to a head component 420.

The handle 410 houses a light emitting device 450. This may be any suitable conventional light emitting device suitable for use in a handheld device. A light guide tube 460 is configured to transport light from the light emitting device to the light emitting structure of the head component for emission to a target area of the user's teeth and gums. In this exemplary embodiment the light emitting device may simply be the terminus of the light guide tube which is embedded within a nozzle 470.

The handle 410 further houses a fluid reservoir 430 which is configured to store fluid suitable for use in a fluid-based cooling system of the head component. The pump 440, also contained within the handle 410, is configured to pump fluid from the fluid reservoir to the fluid-based cooling system. In this embodiment, the fluid-based cooling system comprises the nozzle 470 configured to emit fluid to the target area of an oral surface of the user, and the light guide tube 460 comprises a fluid channel.

The light guide tube 460, contains and directs a fluid flow from the fluid reservoir 430 to the nozzle 470 for emission to the target area. In this way, the light guide tube may serve the dual purpose of establishing the fluid flow and ensuring efficient transfer of light from the light emitting device to the light emitting structure for emission. For this purpose, the light guide tube 460 is made of optical silicon, with a refractive index $n \approx 1.4$ that is close to that of the fluid, but different to that of air. Light is therefore transmitted at the interface between the fluid and the optical silicon but reflected at the interface between the silicon and air, meaning that as long as the silicon tubing within the handle is surrounded by a layer of air, both the light guide tube and the fluid channel contained within it act as a light guide. Other suitable materials, with the correct optical properties may alternatively be used to form the light guide tube 460. Alternatively, any tubing material may be provided with a reflective coating on its inner surface to maximise light reflection at the interface of the tube and thereby enable the water within the tubing to act as a light guide.

The advantage of the configuration shown in Fig. 4, wherein a light emitting device is placed at a location in the handle removed from the portion of the head component that is configured to be adjacent to oral surfaces of the subject when the device is in use, is that heat dissipated by the light emitting device is not absorbed by the subject's oral surfaces. Providing a light emitting device in the handle of the device may also suffer less restrictive space constraints. However, more light losses will be suffered during the transport of light from the light emitting device of the handle to the light emitting structure of the head component.

Although in this embodiment, the fluid channel and light guide tube form one component, in other embodiments they may be implemented separately. For example, a rubber, plastic, or metal fluid channel may be placed adjacent to an optical fibre which acts in the capacity of the light guide tube and directs the light emitted by the light emitting device 450 to the nozzle 470. However, the provision of the fluid channel and light guide tube in one component may be preferable since optical fibres may be more expensive. Additionally, an optical fibre is typically thinner than a fluid channel used as a light guide tube and therefore when an optical fibre is used it may be more difficult to align the light emission from the head component to emit to the target area.

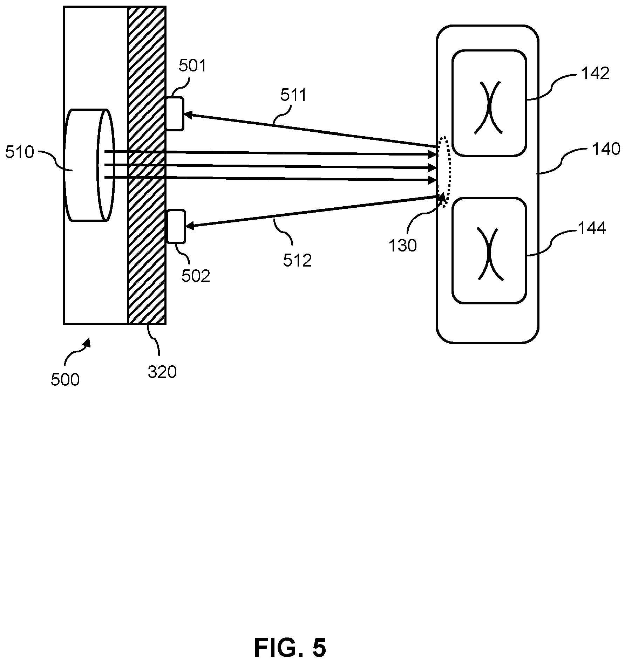

Although Figs. 4 and 5 depict embodiments of the invention in the form of a mouthpiece device and an oral irrigator respectively, the proposed invention is not limited to such products. The concept of the proposed invention, namely light treatment and fluid-based cooling, may be applied to a wide range of oral health care systems and devices.

Referring now to Fig. 5, there is depicted a simplified block diagram of a portion of a head component 500 for an oral care device according to another embodiment.

The head component 500 comprises a light emitting structure 510, configured to emit light to a target area 130 of the teeth and gums of the user, and a fluid-based cooling system 520 configured to establish a fluid flow that extracts heat from the target area.

In this embodiment of the present invention, the head component 500 further comprises the light sensors 501 and 502. Some of the light emitted from the light emitting structure 510 that is incident on the target area 530 may be reflected. Reflected rays 511 and 522 may then be detected by the light sensors 501 and 502 respectively. The intensity of light detected by the sensors 501 and 502 may be indicative of the particular type of oral surface being targeted. For example, gum surfaces may absorb more violet or blue light than tooth surfaces, and stained teeth may absorb more light than unstained teeth. Further the intensity of reflected light may be further indicative of certain oral diseases or health conditions. For example, local gingivitis causes more blood to be present in the gums which will increase their absorbance.

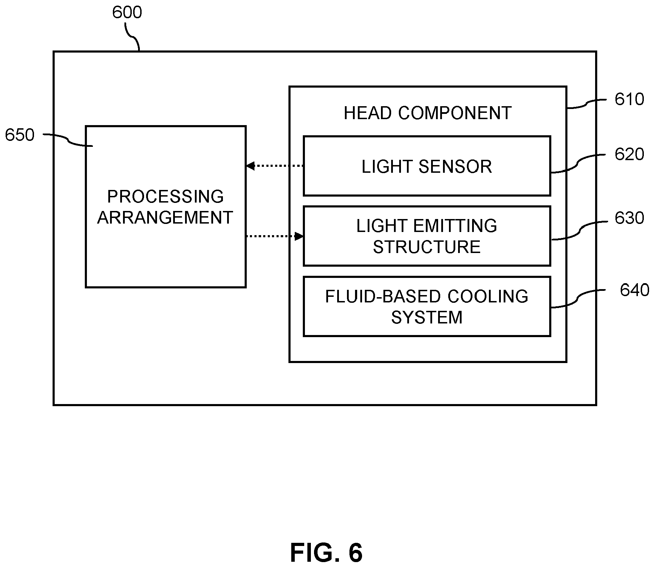

Referring now to Fig. 6, there is depicted a simplified block diagram of an oral care product 600 according to another proposed embodiment.

The oral care product 600 comprises a head component 610. This may be the head component of any proposed embodiment comprising a light emitting structure 630 and a fluid-based cooling system 640. In this exemplary embodiment the head component 610 further comprises at least one light sensor 620. This may be equivalent to light sensors 501 and 502 shown in Fig. 5. The light sensor 620 is configured to generate a light detection signal describing the light reflected off the target area of the subject when the system is in use. For example, the light detection signal may contain information about the intensity of light detected by the light sensor and its wavelength.

The oral care product 600 further comprises a processing arrangement 650 configured to receive the light detection signal from the light sensor 620. Based on this signal the processing arrangement is further configured to control the light emitted by the light emitting structure. In this embodiment, controlling the light emitted by the light emitting structure of the mouthpiece comprises at least one of: controlling the intensity of the light emitted; controlling the power of the light emitted; and controlling the wavelength of the light emitted.

The properties of the light reflected by the target area may be indicative of the type of oral surface that is being treated. This embodiment may therefore allow for control of the light emitting structure into different treatment modes that may be more beneficial for different oral surfaces. Further, the reflected light may indicate areas of plaque or gum inflammation and allow control of the operation of the device to target these areas. In some embodiments, in which the fluid-based cooling system comprises a fluid jet, the processing arrangement may be further configured to control operation of the jet. For example, the orientation and power of the jet may be altered responsive to the light detection signal. Control of the light emitting structure in this way may allow for more effective treatment and improved oral hygiene benefits.

As discussed above, the oral care product 600 makes use of a processing arrangement. The processing arrangement can be implemented in numerous ways, with software and/or hardware, to perform the various functions required. The processing arrangment typically employs one or more microprocessors that may be programmed using software (e.g., microcode) to perform the required functions. The processing arrangement may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processing arrangement may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into the processing arrangment.

**Claims**

1. A head component for an oral care product for insertion into a subject's mouth during use, wherein the head component comprises:

   a light emitting structure (110) configured to emit light to a target area (130) of an oral surface of the subject when in use; and
   a fluid-based cooling system (120) configured to establish a fluid flow that extracts heat from the target area.

2. The head component of claim 1, wherein the fluid-based cooling system (120) comprises a nozzle (124) configured, in use, to emit a jet of fluid (126) to the target area (130), and
   wherein the fluid flow comprises the jet of fluid.

3. The head component of claim 2 wherein the light emitting structure (110) is embedded within the nozzle.

4. The head component of any of claims 2-3, wherein the light emitting structure (110) is located behind the nozzle (124) and wherein the nozzle is transparent.

5. The head component of any preceding claim, wherein the fluid-based cooling system (120) comprises a fluid channel (122) configured to direct the fluid flow.

6. The head component of claim 5, wherein the fluid channel (122) is configured to be adjacent to the teeth and gums of the user when in use.

7. The head component of any of claims 5 and 6, wherein the fluid channel (122) is configured to be adjacent to the light emitting structure (110) so that the fluid-based cooling system (120) is further adapted to extract heat from the light emitting structure.

8. The head component of any of claims 5-7, wherein the fluid channel (122) is configured to transport the fluid flow to a nozzle for emission to the oral surface of the subject.

9. The head component of any of claims 5-8, wherein the fluid channel (122) comprises a closed re-circulation loop within the head component.

10. The head component of any preceding claim, wherein the light emitting structure (110) is configured to emit at least one of: blue light; violet light; infrared light; and ultraviolet light.

11. The head component of any preceding claim further comprising a light sensor (620) configured to detect light reflected off the target area of the subject when the device is in use.

12. An oral care product for insertion into subject's mouth during use, wherein the oral care product comprises the head component of any preceding claim.

13. The oral care product of claim 12, further comprising:

    a fluid reservoir (430); and
    a pump (440) configured to pump fluid from the fluid reservoir to the fluid-based cooling system.

14. The oral care product of any of claims 12 and 13, further comprising:

    a handle configured to attach to the head component;
    a light emitting device situated in the handle; and
    a light guide tube configured to transport light from the light emitting device to the light emitting structure of the head component, and optionally wherein the light guide tube comprises the fluid-based cooling system.

15. The oral care product of any of claims 12-14, further comprising:

    a light sensor of the head component configured to generate a light detection signal describing the light reflected off the target area of the subject when the system is in use; and
    a processing arrangement configured to:

    receive the light detection signal from the light sensor; and
    based on the light detection signal, control the light emitted by the light emitting structure of the head component, and
    optionally wherein controlling the light emitted by the light emitting structure comprises at least one of:

    controlling the intensity of the light emitted;
    controlling the power of the light emitted; and
    controlling the wavelength of the light emitted.

**FIG. 1A**

**FIG. 1B**

**FIG. 2**

**FIG. 3**

FIG. 4

**FIG. 5**

FIG. 6

Europäisches Patentamt

European Patent Office

Office européen des brevets

# EUROPEAN SEARCH REPORT

**Application Number**

EP 24 15 0836

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/239998 A1 (SNYDER CLIFFORD J [US] ET AL) 23 September 2010 (2010-09-23) * paragraphs [0043] - [0045], [0048], [0058] - [0061] - paragraphs [0076] - [0078]; figure * * | 1-15 | INV.<br>A61C1/00<br>A61B18/20<br>A61C19/06<br>A61N5/06 |
| X | US 2023/139440 A1 (NIKINMAA SAKARI [FI] ET AL) 4 May 2023 (2023-05-04) * paragraphs [0077] - [0082], [0113], [0114]; figure * * | 1-15 | |

| | | | TECHNICAL FIELDS SEARCHED (IPC) |
|---|---|---|---|
| | | | A61C<br>A61N<br>A61B |

The present search report has been drawn up for all claims

| Place of search | Date of completion of the search | Examiner |
|---|---|---|
| Munich | 13 June 2024 | Haller, E |

EPO FORM 1503 03.82 (P04C01)

**EP 4 585 182 A1**

## ANNEX TO THE EUROPEAN SEARCH REPORT
## ON EUROPEAN PATENT APPLICATION NO.

EP 24 15 0836

13-06-2024

| Patent document cited in search report | Publication date | Patent family member(s) | | Publication date |
|---|---|---|---|---|
| US 2010239998 A1 | 23-09-2010 | CA | 2756057 A1 | 23-09-2010 |
| | | CN | 202821693 U | 27-03-2013 |
| | | EP | 2408396 A1 | 25-01-2012 |
| | | JP | 5643284 B2 | 17-12-2014 |
| | | JP | 2012521225 A | 13-09-2012 |
| | | KR | 20110139748 A | 29-12-2011 |
| | | KR | 20140009537 A | 22-01-2014 |
| | | MX | 350876 B | 19-09-2017 |
| | | US | 2010239998 A1 | 23-09-2010 |
| | | WO | 2010108189 A1 | 23-09-2010 |
| US 2023139440 A1 | 04-05-2023 | AU | 2020365063 A1 | 05-05-2022 |
| | | CA | 3157705 A1 | 15-04-2021 |
| | | CN | 112641529 A | 13-04-2021 |
| | | EP | 4041381 A1 | 17-08-2022 |
| | | FI | 20195878 A1 | 12-04-2021 |
| | | JP | 2022553650 A | 26-12-2022 |
| | | KR | 20220103712 A | 22-07-2022 |
| | | US | 2023139440 A1 | 04-05-2023 |
| | | WO | 2021069805 A1 | 15-04-2021 |

EPO FORM P0459

For more details about this annex : see Official Journal of the European Patent Office, No. 12/82